Europäisches Patentamt

(19) European Patent Office

Office européen des brevets

(11) Veröffentlichungsnummer: **0 201 525**
**B1**

(12) EUROPÄISCHE PATENTSCHRIFT

(45) Veröffentlichungstag der Patentschrifft:
21.03.90

(21) Anmeldenummer: 85905198.9

(22) Anmeldetag: 29.10.85

(86) Internationale Anmeldenummer:
PCT/CH 85/00158

(87) Internationale Veröffentlichungsnummer:
WO 86/02539 (09.05.86 Gazette 86/10)

(51) Int. Cl.⁵: **A 61 B 5/06**, G 01 V 3/10

(54) VORRICHTUNG ZUR LOKALISIERUNG VON METALLISCHEN GEGENSTAENDEN IM MENSCHLICHEN ODER TIERISCHEN KOERPER.

(30) Priorität: 01.11.84 CH 5221/84

(43) Veröffentlichungstag der Anmeldung:
20.11.86 Patentblatt 86/47

(45) Bekanntmachung des Hinweises auf die Patenterteilung:
21.03.90 Patentblatt 90/12

(84) Bennante Vertragsstaaten:
CH DE FR GB LI

(56) Entgegenhaltungen:
GB-A-994 400
US-A-4 291 280
US-A-4 416 289

(73) Patentinhaber: NYBERG, Pekka Johannes
Spyristrasse 11
CH-8044 Zürich (CH)
Patentinhaber: WEBER, Thomas
Bombachstrasse 22
CH-8049 Zürich (CH)

(72) Erfinder: NYBERG, Pekka Johannes
Spyristrasse 11
CH-8044 Zürich (CH)
Erfinder: WEBER, Thomas
Bombachstrasse 22
CH-8049 Zürich (CH)

(74) Vertreter: Frei, Alexandra Sarah
Frei Patentanwaltsbüro Hedwigsteig 6 Postfach 95
CH-8029 Zürich (CH)

LIBERGRAF, STOCKHOLM 1990

**Beschreibung**

Die Erfindung betrifft ein Gerät gemäss Oberbegriff des Patentanspruchs 1.

Die Lokalisierung von metallischen Gegenständen, die entweder beabsichtigt durch ärztliche Massnahmen oder unfallmässig in den menschlichen oder tierischen Körper gelangen, erfolgt entweder durch Röntgenabbildung oder durch Ultraschalluntersuchung. Zur genauen räumlichen Ortung eines solchen Fremdkörpers werden vor der geplanten operativen Entfernung mehrere gezielte Röntgenaufnahmen angefertigt. Während des Eingriffes sind häufig erneute Röntgenaufnahmen oder Durchleuchtungen, d.h. die Verwendung von Bildverstärkern nötig, insbesondere dann, wenn es sich um kleinere Metallteile handelt. Diese Art röntgenunterstützte Operation setzt den Patienten und das Operationsteam nicht nur einer erhöhten Strahlenbelastung aus, auch die Operation wird durch einen beträchtlichen apparativen Aufwand kompliziert und zeitlich mitunter bis zur Belastungsgrenze für alle Beteiligten ausgedehnt. Nicht selten müssen die Operationen sogar erfolglos abgebrochen werden, da im blutenden Gewebe kleine Metallpartikel optisch unaufspürbar sein können. Um die Traumatisation des Gewebes durch die Operation möglichst gering zu halten, ist eine rasche und sichere Lokalisierung eines gesuchten Fremdkörpers in jedem Falle sehr erwünscht.

Bekannt sind Metalldetektoren, wie sie zum Aufspüren von meist im Boden vergrabenen Metalls vorgesehen sind. Auch sind Metalldetektoren bekannt, mit denen in Wänden Leitungen oder in Betonwänden Eisenarmierungen detektiert werden können. Alle diese Geräte eignen sich jedoch aus verschiedenen Gründen nicht für den Einsatz im Operationssaal. Die in Operationssälen verwendete ausgefeilte, komplizierte und teure Röntgenbildverstärker-Technik und das völlige Fehlen von Metalldetektoren, deutet darauf hin, dass der Einsatz von bekannten Metalldetektoren im Operationssaal ungeeignet sein muss.

Aus der Patentschrift US-4 291 280 ist bspw. eine Schaltung zur Detektion eines Metallkörpers bekannt. Der dort verwendete Oszillator, bei dem es sich um ein fertiges Industrieprodukt handelt, ist für medizinische Anwendungen nicht geeignet, da einerseits die Dimensionen der verwendeten Spule zu gross sind andererseits die Empfindlichkeit der Gesamtvorrichtung ungenügend ist. Eine eigentliche Auswertung des Messignals erfolgt nicht, sondern es entsteht ein "logisches" Ausgangssignal, das im käuflichen Oszillator generiert wird. Damit kann die Vorrichtung nur die Präsenz von Metallteilen anzeigen, jedoch nicht deren Lage. Eine echte Ortung ist nicht möglich. Ausserdem fehlt der Vorrichtung ein automatischer Abgleich. In einem anderen Patent GB-994 400 ist eine Sonde und eine Vorrichtung beschrieben, die der Ortung von Metallteilen im menschlichen oder tierischen Gewebe dienen. Auch diese Vorrichtung erreicht nur eine ungenügende Sensitivität und erfordert einen relativ hohen Schaltungsaufwand.

Es ist Aufgabe der Erfindung eine Vorrichtung zum Lokalisieren von in menschlichen oder tierischem Körper befindlichen Metallgegenständen zu schaffen. Die Vorrichtung soll auch eine einfache Handhabung während der Operation ermöglichen.

Die Aufgabe wird durch die in den Patentansprüchen angegebene Erfindung gelöst.

Eine spezielle Ausführungsform der Erfindung wird nun mit Hilfe der nachfolgend aufgeführten Figuren eingehend diskutiert.

Figur 1 zeigt als bspw. Ausführungsform ein batteriebetriebenes Gerät mit Handsonde, wie es sich für den Einsatz im Operationssall eignet;

Figur 2 zeigt in schematisierter Darstellung einen Sondenkopf der Vorrichtung aus einem Schalenkern bestehend, mit den zugehörigen magnetischen Feldlinien;

Figur 3 zeigt in schematisierter Darstellung einen Sondenkopf der Vorrichtung aus einem Ferritstab bestehend, mit den zugehörigen magnetischen Feldlinien;

Figur 4 zeigt in Blackbox-Darstellung die prinzipielle Schaltung; wie sie in der Vorrichtung gemäss Erfindung verwendet wird;

Figur 5 zeigt im Blockschaltbild eine spezielle Ausführungsform der Beschaltung der Vorrichtung gemäss Erfindung;

Figur 6 zeigt das Schaltbild einer speziellen Ausführungsform des Oszillators gemäss Erfindung.

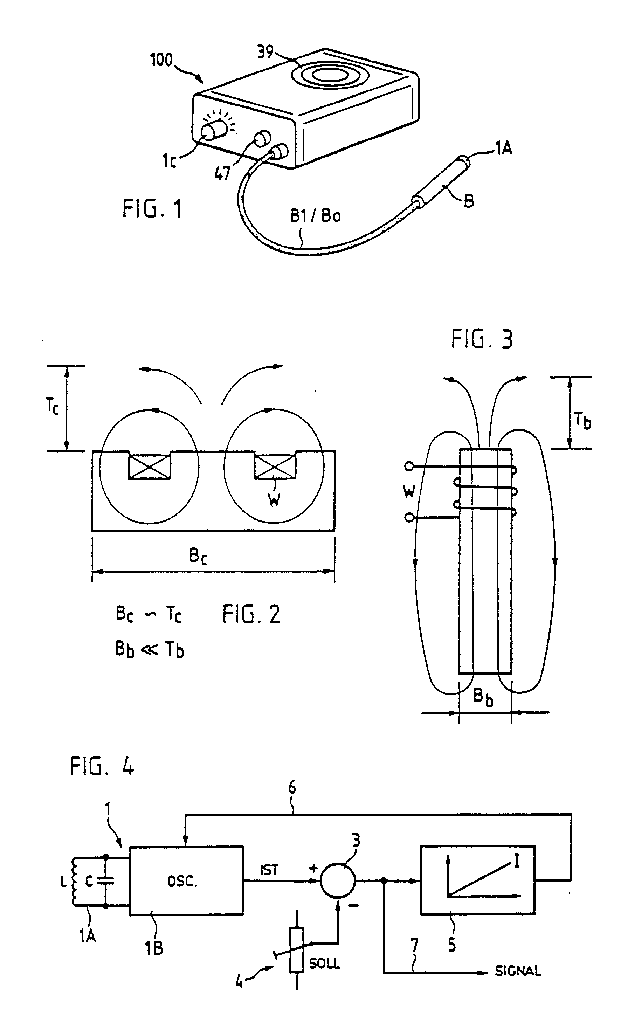

Die physische Erscheinung der Vorrichtung, wie sie Figur 1 zeigt, ist ein elektrisches Schalt- und Angabegerät mit einer an einer dünnen elektrischen Zuleitung B0/B1 angeschlossenen stiftförmigen Sonde, die an ihrem Vorderteil einen "Suchkopf" 1A aufweist. Das Angabegerät ist ein vorzugsweise batteriebetriebenes Kästchen 100, mit wenigen Bedienungselementen 1C, 47 und ebenso vorzugsweise mit einer Signalangabe, die den Benützer visuell nicht in Anspruch nimmt, bspw. einem kleinen Lautsprecher 39 für akustische Signalgebung. So präsentiert sich die Vorrichtung dem Chirurgen, der damit arbeiten soll.

Um diese Bedingungen zu erfüllen, muss die Vorrichtung einen hohen Grad an Selbstbedienung oder Automatik aufweisen, hauptsächlich wird an die Empfindlichkeit, an das Langzeitverhalten und den Abgleich, hohe Anforderungen gestellt. Ausserdem soll die Sonde beim Praktizieren trotz hoher Empfindlichkeit in der Detektion von Metallgegenständen möglichst unempfindlich gegen andere Einflüsse, insbesondere gegen solche menschlichen oder tierischen Gewebes sein, das als elektrolythaltiges Material elektrisch keineswegs indifferent ist, Beispiel EKG, EEG etc..

Die Figuren 2 und 3 zeigen zwei für die Vorrichtung taugliche Sondentypen in stilisierter Darstellung. Die Geometrie der magnetischen Feldlinien ist gegeben durch die Geometrie der ferromagnetischen Spulenkörpern und die Tatsache, dass die magnetischen Feldlinien geschlossen sind.

Figur 2 zeigt die Oszillatorspule W in einen Schalenkern von Durchmesser Bc eingebettet, die mit Pfeilen symbolisierten magnetischen Feldlinien erreichen dabei eine relative Wirkungstiefe von ungefähr Tc. In grober Näherung kann die Wirkungstiefe der Sonde in der Grössenordnung des Schalenkerndurchmessers festgelegt werden. Wird jedoch eine dünne, stiftförmige Sonde bevorzugt, so bringt man die Spule W auf einen Ferritstab auf. Die durch den Ferritstab laufenden Feldlinien, die sich ja nota bene schliessen müssen, erreichen bei wesentlich geringerem Durchmesser Bb der Sonde eine vergleichbare Eindringtiefe Tb wie die Tiefe Tc der Schalenkernsonden, dafür sind sie aber wesentlich länger.

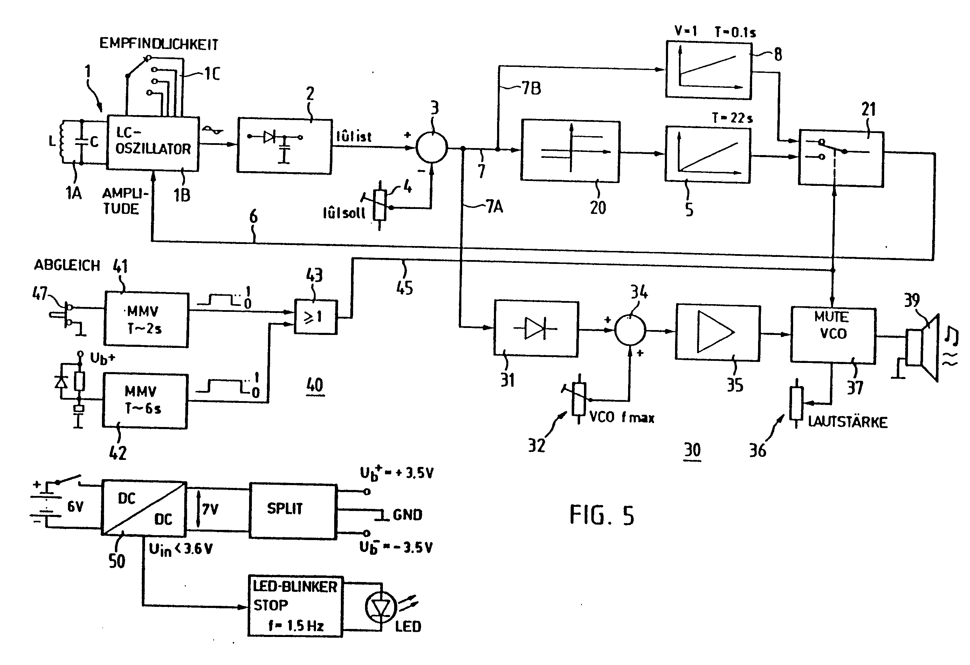

Figur 4 zeigt die Prinzipschaltung der Vorrichtung gemäss Erfindung als Blockschaltbild. Als Ausgangssignal bzw. Regelgrösse dient die Amplitude des Oszillators. Das Ausgangssignal des offenen Oszillators 1 mit einem Schwingkreis 1A und der entsprechenden Schaltung 1B ist als IST-Wert auf einen von zwei Eingängen (+) oder (-) eines Vergleichers 3 geführt. Der andere Eingang des Vergleichers 3 ist mit einem Sollwertgeber 4 für den Amplitudensollwert verbunden. Das Differenzsignal in der Grösse der Regelabweichung ist einerseits das über eine Leitung 7 abgegriffene Nutzsignal und andererseits wird es einem Regelglied 5, hier ein I-Regler zugeführt, dessen Ausgangssignal über eine Leitung 6 auf den Oszillator 1 zurückgeführt ist. Bei einer anderen Ausführungsform kann das auszuwertende Nutzsignal anstatt nach der Vergleichschaltung, an der feed-back-Leitung 6, also nach dem Regler abgezweigt werden. Auf diese Weise kann der Oszillator mit befriedigendem Langzeitverhalten in einem Empfindlichkeitsbereich betrieben werden, bei dem die Oszillatoramplitude gerade noch aufrecht erhalten wird und schon bei Annäherung eines kleinen Metallstückes, das ist die beabsichtigte Störung, die auf den Regelkreis via Oszillator einwirken soll, die Spannung abreisst bzw. die Amplitude zusammenbricht. Der Oszillator mit Sonde ist damit ein Teil einer Regelschaltung, die eingehender in Figur 5 dargestellt ist.

Diese Figur 5 zeigt ebenfalls im Blockschaltbild eine spezielle Ausführungsform der Erfindung. Auf eine spezielle Ausführungsform des Oszillators wird später eingegangen. Der Oszillator 1 mit dem den Schwingkreis tragenden Sondenteil 1A und der Verstärkungsschaltung 1B ist über einen Mehrfachschalter 1C auf eine Mehrzahl von Empfindlichkeitsstufen einstellbar. Das Ausgangssignal in der Grössenordnung 2Vpp/200kHz wird auf einen Gleichrichter 2 mit Tiefpasswirkung geführt, dessen Ausgang die Amplitude des Oszillators als IST-Wert abgibt. Im Vergleicher 3 entsteht die Regelabweichung zum Sollwert, die naturgemäss beide Vorzeichen aufweisen kann. Sie wird am Ausgang der Schaltung auf die Leitung 7 abgegeben. Über eine verzweigte Leitung 7A wird das Nutzsignal abgeführt und über eine weitere verzweigte Leitung 7B ist der Eingang einer alternativ zu betreibenden Regelschaltung 8 angeschlossen. Die Regelabweichung wird einer Begrenzerschaltung 20 zugeführt, welche eine Gewichtung der positiven und negativen Regelabweichung bewirkt. Das Verhältnis beträgt hier etwa 10 : 1 zugunsten der positiven Regelabweichung. Die vom Begrenzer 20 abgegebene Spannung wird in einem I-Regelglied integriert. Die Zeitkonstante soll für diesen Anwendungszweck 5 bis 30 Sekunden betragen. Der integrierte Spannungswert wird schliesslich über die Leitung 6 als Stellgrösse auf den Oszillator zurückgeführt.

Dem eben beschriebenen Regelkreis sind noch zwei weitere Schaltungen zugeordnet, nämlich eine spezielle Ausführungsform einer Nutzsignal-Auswerteschaltung 30 und eine auf den Regelkreis wirkende Abgleichschaltung 40.

Das Auswertesignal wird für den eingangs genannten klinischen Zweck in der Schaltung 30 akustisch umgesetzt. In dieser Ausführungsform wird dazu ein spannungsgesteuerter Oszillator 37 (voltage controlled oscillator VCO) verwendet, dessen Freilauffrequenz VCOfmax über den verstellbaren Abgriff 32 in den Audiobereich gelegt wird. Das auf der Leitung 7A anstehende Nutzsignal wird im Gleichrichter 31 gleichgerichtet und einem der Eingänge einer Addierschaltung 34 zugeführt, ein anderer Eingang erhält das Signal VCOfmax. Das Summensignal wird in einem Verstärker 35 verstärkt und schliesslich an den VCO abgegeben. Der VCO setzt die sich als Amplitudenänderung manifestierende Bedämpfung des Oszillators in eine Frequenzänderung um. Das heisst, bei Annäherung an einen metallischen Körper nimmt die nehmen Frequenz (des VCO, nicht des Sondenoszillators) kontinuierlich ab und beim Entfernen wieder zu.

Da vorzugsweise für das Gerät, das grosse bis hinunter zu sehr kleinen Metallgegenständen mit jeweils höchster Empfindlichkeit detektieren soll, verschiedene Empfindlichkeitsstufen vorgesehen sind -der Oszillator wird erfindungsgemäss durch den Regelkreis gerade noch am Schwingen gehalten, sodass schon ein verhältnismässig kleines Metallteil die Schwingung abreisst- soll der Regelkreis einfach und leicht auf jeden Empfindlichkeitsgrad abgleichbar sein. Dafür ist eine Abgleichschaltung 40 vorgesehen, die aus zwei Univibratoren (Oneshot) 41, 42 mit verschiedenen Zeitkonstanten T, einem logischen Gatter OR 43, das mit einem dazugehörenden elektronischen Schalter 21 verbunden ist, sowie mit einem raschen, zuschaltbaren PI-Regelglied 8 im Hauptregelkreis. Auf dem durch das OR-Gatter 43 geschlauften Signalpfad gelangen die Univibratorsignale auf die Leitung 45, mit welchen Signalen der elektronische Schalter entsprechend den Zeitkonstanten geschaltet ist. Die Schaltung ist bspw. so ausgelegt, dass beim Drücken des Abgleichtasters 47 für ca. zwei Sekunden der langsame I-Regler weggeschaltet und der schnelle (Abgleich-) PI-Regler zugeschaltet wird. Simultan dazu wird der VCO stumm geschaltet (muting), da bei diesem schnellen Abgleich der Kleinlautsprecher zu heulen begänne.

Vorzugsweise ist das Detektiergerät batteriegespiesen. Dazu eignet sich der in Figur 5 abgebildete Netzteil mit dem DC/DC-Wandlerbaustein RC4193 von Raytheon.

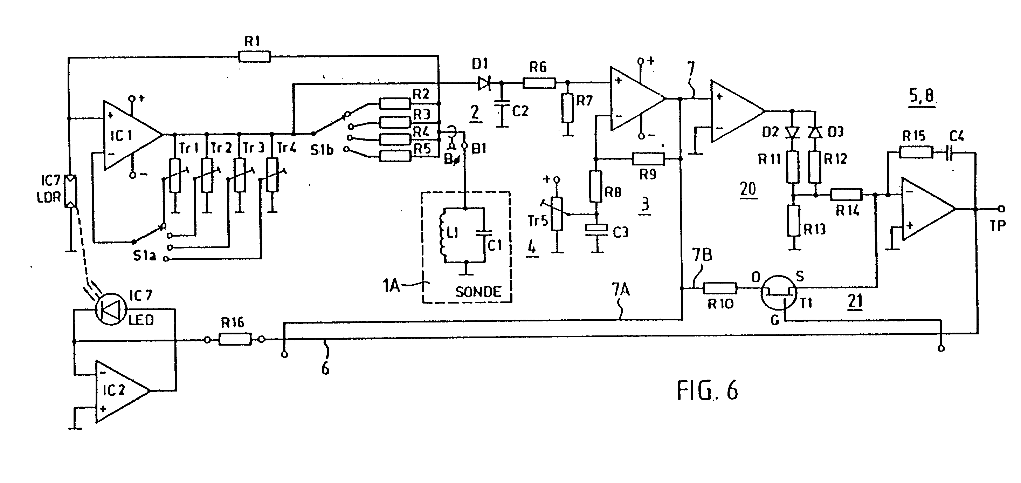

Eine bevorzugte Ausführungsform des Oszillators ist in Figur 6 gezeigt und zwar betrifft dies speziell die Rückkopplung des Regelkreises zum Oszillator. Zur Verhinderung einer unerwünschten Rückwirkung vom Ausgang auf den Eingang der Einkopplung vom Regelkreis auf den Oszillator, d.h. auf das Einkopplungsnetzwerk, wird an Stelle einer galvanischen Verbindung eine optische Ankopplung an den Oszillator verwendet. Der in der Sonde untergebrachte Schwingkreis 1A mit den Bauelementen L1 und C1 ist für den vorgesehenen Zweck auf eine Frequenz von 150 kHz bis 500 kHz ausgelegt. Da die Sonde entsprechend ihrem Bestimmungszweck mit lebenswarmem Gewebe zeitweilig in Berührung kommt, wird für die induktive Wicklung an Stelle von Kupfer ein Material mit einem geringeren Temperatur/Widerstandskoeffizienten verwendet, wie bspw. Konstantan etc., um den mit einer Zeitkonstante von T = 10 bis 35 s relativ langsam eingestellten Regler, der wie gesagt den Oszillator nahe an einem seiner Grenzbereiche eher "kritisch" betreibt, nicht zu überfordern.

Beginnend bei der optischen Rückkopplung mit einem Photosender und einem Photoempfänger, wird zur Realisierung des Senders das aus dem Regler 5 bzw. 8 rückgeführte Signal auf der Leitung 6 in einer Photodiode LED umgesetzt. Der Operationsverstärker IC2 ist mit dem Widerstand R16 als spannungsgesteuerte Stromquelle für die Photodiode LED geschaltet. Die variable Leuchtdichte wird von einem photosensitiven Widerstand LDR aufgenommen und steuert den Grad der Mitkopplung des Operationsverstärkers IC1. Das Gegenkopplungsnetzwerk ist durch eine Anzahl variabler Spannungsteiler Tr1 bis Tr4 realisiert. Über die umschaltbaren Seriewiderstände R2 bis R5 kann der Schwingkreis mehr oder weniger lose angekoppelt werden. Die erwähnten Widerstände werden bspw. über einen Mehrebenenschalter S1a/S1b miteinander umgeschaltet. Richtwerte dieser Oszillatorschaltung sind: LDR > R1 wobei LDR 5 – 500 kOhm und R1 100 – 50 Ohm. Die Empfindlichkeitsabstufung kann beginnen mit Tr1 ca. 5 kOhm/R2 – R5 50 – 1000 Ohm, wobei in der genannten Grössenordnung Tr2 bis Tr4 nach Wunsch und Bedarf ausgelegt sein kann. Die Trimmer Tr1 – 4 werden beim Zusammenbau einmal abgeglichen, es sind also reine Einstell- und keine Bedienungsmittel. Der Schwingkreis, das heisst die Induktivität und die Kapazität, sind in der Handsonde untergebracht, welche mit einem abgeschirmten Koaxkabel mit der Regelschaltung verbunden ist.

Wenn, wie gesagt der Oszillator "kritisch" am Rande seiner Schwingfähigkeit betrieben wird und eine relativ geringe Störung des Oszillators die Schwingung abreissen lässt, so empfiehlt es sich, die den Schwingkreis tragende Handsonde gegen seitliche Störwirkungen, im Gegensatz zur gewollten stirnseitigen Einwirkung, zu schützen. Dazu wird vorzugsweise die Sonde durch eine enge, sich über ihre gesamte Länge erstreckende Drahtwicklung abgeschirmt, welche an Masse gelegt ist. So lässt sich die Sonde manipulieren, ohne dass der Regelkreis beeinflusst wird.

## Patentansprüche

1. Vorrichtung zur Lokalisierung von Metallgegenständen im menschlichen oder tierischen Körper mit folgender Prinzipschaltung: der Ausgang eines Oszillators (1) ist mit einem der Eingänge einer SOLL/IST-Wert-Vergleichsschaltung (3) verbunden, deren Ausgang mit dem Eingang einer Regelschaltung (5 und/oder 8) verbunden ist und der Ausgang der Regelschaltung (5, 8) ist auf den Oszillator (1) zurückgeführt, dessen Ausgangsspannungsamplitude den Ist-Wert liefert, wobei eine mit dem Oszillator (1) verbundene Induktionsspule (L) einer Messonde (1A) den Oszillator so beeinflusst, dass die Oszillatorspannung bei Annäherung eines Metallstückes abreisst, dadurch gekennzeichnet, dass das Nutzsignal am Ausgang der Vergleichsschaltung (3) oder am Ausgang der Regelschaltung (5, 8) zur Weiterverarbeitung abgezweigt ist und dass die Induktionsspule (L1) der Messonde (1A) aus einem Material mit geringerem Temperatur/Widerstands-Koeffizienten als Kupfer gefertigt ist.

2. Vorrichtung nach Anspruch 1, dadurch gekennzeichnet, dass das Spulenmaterial ein Konstantandraht ist.

3. Vorrichtung nach einem der Ansprüche 1 oder 2, dadurch gekennzeichnet, dass die Regelschaltung mindestens zwei parallelgeschaltete und mittels einem Schalter (21) alternativ zuschaltbare Regler (5, 8) mit je verschiedenen Zeitkonstanten und Regelcharakteristiken enthält.

4. Vorrichtung nach Anspruch 3, dadurch gekennzeichnet, dass die Regelschaltung einen I-Regler mit Zeitkonstante im Sekundenbereich (5) für den Betrieb und ein PI-Regler und Zeitkonstante im Subsekundenbereich (8) für den Abgleich enthält.

## Claims

1. Instrument for locating metal objects in the human or animal body with the following circuitry: the output of an oscillator (1) is connected to one of the inputs of a desired/actual value comparator (3), whose output is connected to the input of a control circuit (5 and/or 8) and the output of the latter is returned to the oscillator (1), whose output voltage amplitude supplies the actual value, an induction coil (L) of a probe (1A) connected to the oscillator (1) influencing the latter in such a way that on approaching a metal object the oscillator voltage is interrupted, characterized in that at the output of the comparator (3) or at the output of the control loop (5, 8), the information signal is branched off for further processing and that the induction coil (L) of probe (1A) is produced from a material having a lower temperature/resistance coefficient than copper.

2. Instrument according to claim 1, characterized in that the coil material is a constantan wire.

3. Instrument according to one of the claims 1 or 2, characterized in that the control loop contains at least two parallel-connected controllers (5, 8), which can be alternately connected in by means of a switch (21) and having in each case different time constants and control characteristics.

4. Instrument according to claim 3, characterized in that the control circuit contains an I-controller with a time constant in the second range (5) for operation and a PI-controller and time constant in the subsecond range (8) for the balancing.

**Revendications**

1. Dispositif pour localiser des objets métalliques dans le corps humain ou animal comprenant le circuit de principe suivant: la sortie d'un oscillateur (1) est reliée à l'une des entrées d'un circuit comparateur valeur de consigne/valeur réelle (3), dont la sortie est reliée à l'entrée d'un circuit régulateur (5 et/ou 8) et la sortie du circuit régulateur (5, 8) est connectée à l'oscillateur (1) dont l'amplitude de tension de sortie donne la valeur réelle, une bobine d'induction (L) reliée à l'oscillateur (1) d'une sonde de mesure influence l'oscillateur, de sorte que la tension de l'oscillateur varie brusquement à l'approche d'une pièce métallique, caractérisé en ce que le signal utile à la sortie du circuit régulateur (5, 8) est dirigé vers le traitement et que la bobine d'induction (L1) de la sonde de mesure (1A) est réalisée en un matériau de coefficient de température résistant inférieur à celui du cuivre.

2. Dispositif selon la revendication 1, caractérisé en ce que le matériau de la bobine est un fil en constantan.

3. Dispositif selon l'une des revendications 1 ou 2, caractérisé en ce que le circuit régulateur comprend au moins deux régulateurs branchés en parallèle (5, 8) et interconnectés alternativement au moyen d'un contacteur (21), ceux-ci ayant des constantes de temps et des caractéristiques de réglage différentes.

4. Dispositif selon la revendication 3, caractérisé en ce que le circuit de réglage comprend un régulateur de type I de constante de temps de l'ordre de la seconde (5) pour le fonctionnement et un régulateur de type PI de constante de temps de l'ordre de la seconde (8) pour l'équilibrage.

FIG. 1

$B_c \sim T_c$    FIG. 2

$B_b \ll T_b$

FIG. 3

FIG. 4

SIGNAL

FIG. 5

FIG. 6